# EUROPEAN PATENT SPECIFICATION

(11) **EP 2 721 411 B1**
(45) Date of publication and mention of the grant of the patent: **18.04.2018**
(21) Application number: 12729548.3
(22) Date of filing: 19.06.2012
(51) Int. Cl.: G01N 33/533, G01N 33/68

(54) **A METHOD FOR DETECTING AND/OR QUANTIFYING CARBONYLATED PROTEINS**
VERFAHREN ZUM NACHWEIS UND/ODER ZUR QUANTIFIZIERUNG VON CARBONYLIERTEN PROTEINEN
PROCÉDÉ DE DÉTECTION ET/OU DE QUANTIFICATION DES PROTÉINES CARBONYLÉES

(30) Priority: 20.06.2011 EP 11305772
(43) Date of publication of application: 23.04.2014
(73) Proprietor: Université Pierre et Marie Curie (Paris 6), 75005 Paris (FR)
(72) Inventor: FRIGUET, Bertrand, F-75013 Paris (FR); BARAIBAR, Martin, F-75012 Paris (FR); LADOUCE, Romain, F-75012 Paris (FR)
(74) Representative: Lebrette, Camille
(86) International application number: PCT/EP2012/061749
(87) International publication number: WO 2012/175519

(56) References cited:
- SAWADA, AKIRA ET AL: "Protein carbonyl detection by two-dimensional fluorescence difference gel electrophoresis", JOURNAL OF ELECTROPHORESIS, vol. 52, 15 March 2008 (2008-03-15), pages 9-17, XP002664501, cited in the application
- OIKAWA S ET AL: "Proteomic identification of carbonylated proteins in the monkey hippocampus after ischemia-reperfusion", FREE RADICAL BIOLOGY AND MEDICINE, ELSEVIER SCIENCE, US, vol. 46, no. 11, 1 June 2009 (2009-06-01), pages 1472-1477, XP026086288, ISSN: 0891-5849, DOI: 10.1016/J.FREERADBIOMED.2009.02.029 [retrieved on 2009-03-09]
- MASAMICHI OH-ISHI ET AL: "Proteomic method detects oxidatively induced protein carbonyls in muscles of a diabetes model Otsuka Long-evans Tokushima Fatty (OLETF) rat", FREE RADICAL BIOLOGY & MEDICINE, vol. 34, no. 1, 1 January 2003 (2003-01-01), pages 11-22, XP055013047, ISSN: 0891-5849, DOI: 10.1016/S0891-5849(02)01239-X

## Description

The invention relates to a method for selectively labeling, detecting and/or quantifying protein carbonyl groups in a protein-containing sample, by using one or two-dimensional gel electrophoresis. The method offers high specificity for the detection and/or quantification of protein carbonyl groups, and a means for site-specific identification of protein carbonylation events.

### BACKGROUND OF THE INVENTION

Oxidative stress is involved in several diseases and disorders such as atherosclerosis, cancer, diabetes and Alzheimer's disease. Intermediates or by-products of oxidative stress modify many biomolecules, including proteins.
Protein oxidation has many biochemical consequences, like loss or gain of enzyme activity, loss of protein function, loss of protease inhibitor activity (e.g. α-1-antitrypsin, α-2-macroglobulin), protein aggregation (e.g. prion protein, immunoglobulin), enhanced susceptibility to proteolysis (e.g. glutamine synthetase), abnormal cellular uptake (e.g. LDL), modified gene transcription (e.g. IkB) and increased immunogenicity (e.g. ovalbumin).
Different alterations of proteins are detectable. Among these, protein carbonyls, formed by a variety of oxidative mechanisms, are sensitive indices and the most general and commonly used biomarkers of oxidative injury. Carbonyl groups (aldehydes and ketones) are produced on protein side chains (especially of Proline, Argine, Lysine, and Threonine) when they are oxidized. Chemical reagents (such as reactive oxygen species, H₂O₂, hypochlorous acid, ozone and singlet oxygen, upon others), γ-irradiation, UV light, oxidoreductase enzymes (such as xanthine oxidase, P-450), drugs and their metabolites are known agents that lead to protein carbonyl formation. In addition, carbonyl groups may be introduced into proteins by secondary reaction of the nucleophilic side chains of Cysteine, Histidine, and Lysine residues, with aldehydes (4-hydroxy-2-nonenal, malondialdehyde, 2-propenal [acrolein]) produced during lipid peroxidation or with reactive carbonyl derivatives (ketoamines, ketoaldehydes, deoxyosones) generated as a consequence of the reaction of reducing sugars, or their oxidation products (glycation and glycoxidation reactions), with the eventual formation of the advanced glycation/lipoxidation end products (AGEs/ALEs).

Because protein carbonyls have no distinguishing ultraviolet or visible spectrophotometric absorbance/fluorescence properties, they cannot be directly determined. Instead, detection and quantification of proteins carbonyls require the use of specific chemical probes that serve as handles for determination.
A conventional method for detecting protein carbonyls on a gel-based analysis includes the derivatization of carbonyl groups and subsequently detecting the derivatized molecule through antibody immunodetection. For example, hydrazines, such as DNPH (2,4-dinitrophenyl hydrazine) derivatize protein carbonyls to form corresponding hydrazones, such as dinitrophenylhydrazone (DNP) (Levine et al. "Determination of carbonyl content in oxidatively modified proteins" 1990, Methods Enzymol, 186:464-478 and Winterbourn et al. 1999). This labeling step must be followed first by a separation step by regular one or two-dimensional polyacrylamide gel electrophoresis (ID or 2D-PAGE) and then by a Western blot immunoassay. With such regular 1D or 2D-PAGE a single sample per gel is resolved.
Although this technique has been widely used and successfully applied in a variety of biological systems, several technical limitations exist. Because of subtle changes in experimental conditions, the protein carbonylation patterns on a single Western blot after 2D-PAGE usually cannot be fully duplicated, which makes it difficult to find the carbonylated proteins changed between samples and gels in order to quantify their changes. Although a comparison of protein carbonylation profiles from Western blot after 2D-PAGE can be carried out with the assistance of various software programs, it typically requires some computerized justification of two-dimensional Western blot images so that two images can be superimposed and compared. These difficulties limit the speed and accuracy of quantitation of protein carbonylation after two-dimensional Western blot. In addition, the method is time consuming, employs several reagents and the issue of using antibodies to detect the derivatized product do not assure the specificity required. Furthermore it is rather laborious since only one sample can be separated on each gel.

Sawada et al., (2008, J. Electrophoresis, 52:9-17), performed a preliminary attempt to evidence carbonylated proteins in a single sample by labeling carbonyl groups with a hydrazide-derivatized fluorescent dye after separation by 2D-PAGE. Proteins were labeled with a cyanine fluorophore with an hydrazide functionality reactive with protein carbonyls (CyHz) or CyDye™NHS (the N-hydroxyl succinimidyl ester derivative of a cyanine dye reactive with the ε-amino group of lysine residues). However, Sawada et al. failed to evidence and identify carbonylated proteins at the proteome level.

There is thus a need for an improved method for detecting and/or quantifying carbonylated proteins in a biological sample.

### SUMMARY OF THE INVENTION

In accordance with the present invention, a new method has been developed to label carbonyl groups of carbonylated proteins in at least one protein-containing sample, before performing gel electrophoresis.
The invention provides a method for detecting and/or quantifying protein carbonyl groups in at least one protein-containing sample comprising the steps of subjecting the sample to a denaturing agent that is carbonyl-free, selected from guanidine and salts thereof, labeling the potential protein carbonyl groups with a fluorescent agent before running one dimensional or two-dimensional gel electrophoresis. In specific embodiments, after separating two different samples in the same gel, relative differences in carbonylation between the samples can then be quantified using fluorescent imaging technology.

According to an embodiment of the method of the invention, the resolution of the carbonyl groups may be improved, forming distinct spots that can be analyzed for quantification after two-dimensional differential in gel electrophoresis (2D-DIGE) and further identification by mass spectrometry.

Accordingly, the invention provides a method for detecting and/or quantifying protein carbonyl groups in at least one protein-containing sample comprising the steps of:
(a) subjecting the sample to a protein denaturing agent;
(b) treating the sample with at least a fluorescent agent that is a hydrazide coupled to a fluorescent dye, whereby protein carbonyl groups are labeled;
(c) subjecting the sample to one-dimensional or two-dimensional gel electrophoresis; and
(d) detecting and/or quantifying the labeled carbonyl groups of the proteins resolved by electrophoresis;
wherein the protein denaturing agent is a carbonyl-free denaturing agent selected from guanidine and salt thereof, and is at a concentration between 6M and 8M.

Preferably, the carbonyl-free denaturing agent is guanidinium hydrochloride or guanidine thiocyanate.

An additional step may be performed before step (c) of reducing the Schiff base, or hydrazone, formed by the hydrazide with the protein carbonyl groups. Such additional step stabilizes the labeled carbonylated proteins. Preferably, this stabilization step is performed by using a reducing reagent such as sodium cyanoborohydride, and/or sodium borohydride and/or other salt thereof.

According to the invention, steps (a) and (b) can be simultaneous or subsequent. According to the invention, the stabilization step can be simultaneous or subsequent to step (b).

The method according to the invention can further comprise a step of removing the carbonyl-free denaturing agent, and/or fluorescent agent, and/ reducing agent that remains free or that didn't react, between step (b) and step (c). For example, said removal is performed by size-exclusion chromatography or dialysis.

The method of the invention can further comprise a step of removing DNA, lipids and/or salt residues before step (b), preferably by precipitating the proteins.

Advantageously, at least two samples are separately subjected to differential labeling with at least two fluorescent agents according to step (b) and separated in the same gel.

Relative differences between the samples can then be quantified using fluorescent imaging technology.

In one embodiment of the invention, one of the samples is a standard sample, which is preferably a pooled-sample mixture of all the samples. Said pooled-sample mixture can be prepared by mixing equal protein amounts of all the samples included in the analysis and then labeled with another fluorescent agent suitable for labeling carbonyl groups.

This invention also relates to a method for detecting, quantifying, and/or identifying a protein that becomes carbonylated upon ageing or during a disease, through a mutation or after a treatment, in a biological protein-containing test sample, which method comprises the steps of:
(a1) subjecting the test sample to a protein denaturing agent that is carbonyl-free;
(b1) treating the test sample with a fluorescent agent that is a hydrazide coupled with a fluorescent dye whereby protein carbonyl groups are labeled;
separately
(a2) subjecting a reference or control sample to a protein denaturing agent that is carbonyl-free;
(b2) treating the reference or control sample with a different fluorescent agent that produces a different color fluorescence, whereby protein carbonyl groups are labeled;
(c) mixing the two samples and subjecting them to a two-dimensional differential in gel electrophoresis; and
(d) detecting and/or quantifying the differentially labeled carbonyl groups of the proteins resolved by electrophoresis;
(e) optionally identifying the differentially carbonylated proteins, preferably by using mass spectrometer
wherein the protein denaturing agent is a carbonyl-free denaturing agent selected from guanidine and salt thereof, and is at a concentration between 6M and 8M.

This method may further comprise an additional step of reducing the Schiff base, or hydrazone, formed by the hydrazide with protein carbonyl groups, before step (c), in at least one of the samples, to stabilize the labeled carbonylated proteins.

### BRIEF DESCRIPTION OF THE FIGURES

**Figure 1****.** Detailed chemistry which may be involved in the embodiment of the invention A) Labeling step, and B) Stabilization step;

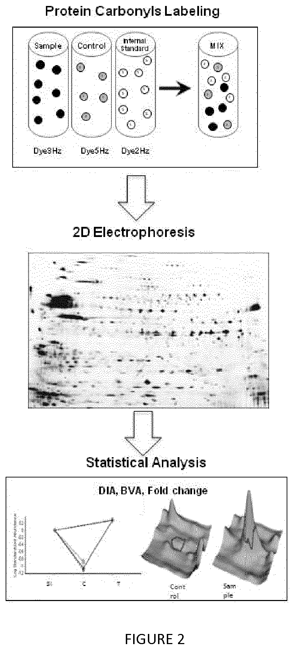

**Figure 2****.** Flow chart of a method of detection and quantification of protein carbonyl groups in protein-containing samples according to an embodiment of the invention, using 2D-DIGE technology;
**Figure 3****.** 1D SDS-PAGE pattern of protein carbonyl groups derived from protein extracts of young (CPD 25) or senescent WI38 fibroblasts, according to an embodiment of the method of the invention, where protein carbonyls are labeled with Cy3Hz, as an example.
**Figure 4****.** Representative gels images of senescent and young WI38 fibroblasts after 2D-DIGE according to an embodiment of the invention, wherein after labeling, proteins derived from senescent and young fibroblasts were subjected to 2D-DIGE. A) Combined protein profile (merge) of young cells, senescent cells and the internal standard; B) Dye2-Hz image of carbonylated proteins from young cells; C) Dye3-Hz image of carbonylated proteins from senescent cells; D) Dye5-Hz image of carbonylated proteins in the internal standard.
**Figure 5****.** Histogram obtained from the differentially in gel analysis representing the distribution of the normalized ratio for each carbonylated spot (discontinuous curve) and a theoretical curve (fitted curve). Volume ratio is displayed on the X-axis and the number of spots in the Y-axis.
**Figure 6****.** A) Representative gel image of senescent and young WI38 fibroblasts after 2D-DIGE according to an embodiment of the invention, with some individual or train of spots identified as differentially carbonylated in young and senescent cells; B) For each spot, 3D representation for each condition is shown as well as the spot volume fold change (Senescent/Control).
**Figure 7****.** A) Representative gel image of human fibroblasts WI38 upon oxidative stress after 2D-DIGE according to an embodiment of the invention, comparing the pattern of carbonylated proteins in black (labeled with Cy3Hz) or total proteins in dark grey (labeled with Cy5NHS). Carbonylated protein spots were further identified by MALDI-TOF/TOF-MS analysis; B) Representative spots trains evidenced as increasingly carbonylated. Arrows indicate those spots that correspond to the same protein identified.

### DETAILED DESCRIPTION

### Definitions

As used herein, a **"protein-containing sample"** refers to any sample to test for the presence of protein carbonyl groups. The sample may comprise a mixture of undefined proteins, or it may be a mixture of predetermined proteins, e.g. prepared in vitro. In a preferred embodiment, the protein-containing sample is a biological sample, i.e. a biological fluid or tissue, or part thereof, or cells.

A **"biological sample"** refers to any biological sample which may contain proteins. Biological samples can originate from any organism, including humans, non-human animals, plants. Examples of biological fluids include blood, urine, plasma, serum, saliva, semen, stool, sputum, cerebral spinal fluid, tears, mucus, amniotic fluid or the like. Examples of biological tissues also include organs, tumors, lymph nodes, arteries, and individual cell(s) (e.g. peripheral blood mononuclear cells).
In the context of the invention, a **"reference sample"** or a **"control sample"** may be a protein-containing sample that has not been subjected to oxidative damage. It may also be a protein-containing sample to compare with the test sample, for detection or quantification of protein carbonylation. For instance when the test sample is a biological sample from a human or animal subject affected with a disease, the reference or control sample may be a biological sample from a healthy subject. When the test sample is a biological sample from a human or animal subject with a mutation at the proteomic or genomic level, the reference or control sample may be a wild-type sample. Samples from the same subject at different times may also be used, for instance to follow up of a therapeutic approach at different stages of the treatment. Advantageously, the reference or control sample is of the same type as the protein-containing sample to test, e.g. the reference or control sample is a blood sample when the test sample is blood.

Alternatively, the reference or control sample for comparison may belong to other sources such as biological fluids (serum, urine, or cerebrospinal fluid) or any kind of biopsies (skin, muscle, etc).

In the context of the invention, the **two dimensional differential in gel electrophoresis (2D-DIGE)** is a variation of the regular 2D-PAGE technology. In the 2D-DIGE technology, specific residues on proteins of at least two different samples are labelled with different fluorescent dyes before to be co-resolved in the same two-dimensional gel. In some embodiment, an internal standard with a third different dye may be co-resolved with the samples on each gel (Unlu, M., Morgan, M. E., and Minden, J. S. (1997) Difference gel electrophoresis: a single gel method for detecting changes in protein extracts. Electrophoresis 18, 2071-2077). The 2D-DIGE may improve reproducibility and accuracy when compared to 2D-PAGE. The incorporation of the internal standard may improve the reliability of the quantitative comparison of the spots between gels (Alban A et al, Proteomics 2003).

### Denaturing step

The disclosure makes use of a protein denaturing agent that is carbonyl-free. The agent is able to denature the proteins, thereby promoting their solubility. The denaturing agent does not comprise any carbonyl group. For example, the denaturing agent is potassium or sodium thiocyanate or litium chloride. In a preferred embodiment, the denaturing agent is guanidine and/or salt thereof, such as guanidinium hydrochloride or guanidine thiocyanate.
The denaturing agent may be comprised in a buffer, such as an extraction or homogenization buffer.
In order to avoid competition with carbonyls in proteins during the step of labeling, the buffer is preferably devoid of any compound that comprises carbonyls. Consequently, according to the invention, the sample is preferably not contacted with urea or CHAPS (3-[(3-Cholamidopropyl)dimethylammonio]-1-propanesulfonate).
However, the sample in denaturing step (a) may be contacted with a buffer that comprises thiourea, preferably between 1M to 3M, more preferably 2M.

The denaturing agent used in step (a) is at a concentration between 6M to 8M, preferably 8M.
In a preferred embodiment, cell pellets or tissue samples are homogenized in a corresponding volume of an extraction buffer comprising guanidinium hydrochloride (preferably 8M) and thiourea (preferably 2M).
The method of the invention may further comprise a step of removing DNA, lipids and/or salt residues of the sample before step (b), preferably by precipitating the proteins. Such an additional step can be useful with tissue or cells sample to avoid non-specific reactions of the fluorescent agent with other molecules present in the tissue or cell extract during the labeling step.
The method of the invention can further comprise a step of adjusting the sample pH to 7 before step (b), in order to optimize the labeling step.
For instance, the denaturing step, and more generally the preparation of the protein extract may be performed as described in Example 1.

### Labeling step

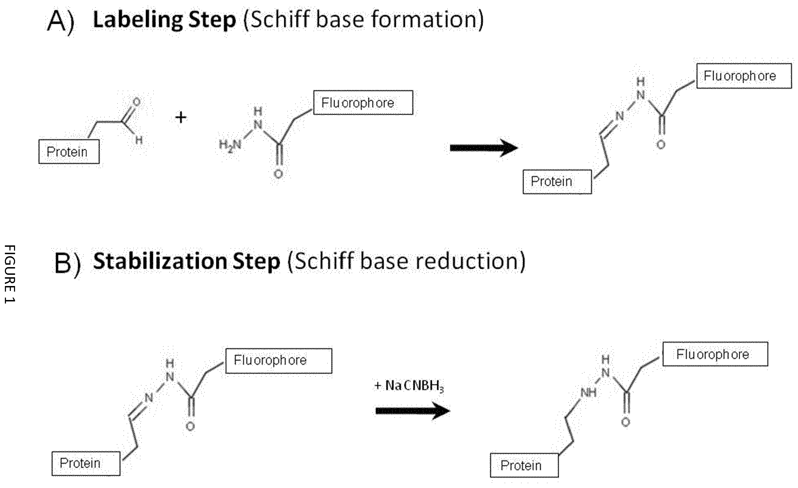

The labeling step according to the method of the invention is conducted by subjecting the protein-containing sample resulting of step (a), with a fluorescent agent that specifically labels carbonyl groups in the proteins of the sample (Figure 1A).
According to the invention, the fluorescent agent is a hydrazide coupled to a fluorescent dye (DyHz). The term "coupled" means that the hydrazide binds the fluorescent dye in such a way that the hydrazide functionality is maintained. Such fluorescent dye with a hydrazide reactive group reacts with the potential protein carbonyl groups in the sample to form a hydrazone, or Schiff base.
The term "specifically" means that the fluorescent agent preferentially binds carbonyl groups, and advantageously exclusively binds carbonyl groups.
Fluorescent agents of the invention include all hydrazide derivatives of a cyanine dye (herein CyHz). For example, the fluorescent agent is Cy-hydrazide dye, such as HiLyte, Fluor® Dyes or Alexa Fluor® Dyes. Said cyanine dyes have generally the same ionic and pH characteristics but absorb and/or fluoresce light at different wavelengths, producing different color fluorescence. For example, Cy3Hz presents a weight of 765.95, a maximum absorbance of 550 nm and a maximum emission of 570 nm. Cy5Hz presents a weight of 791.99, a maximum absorbance of 649 nm and a maximum emission of 670 nm.
For instance, the labeling step may be performed as described in Example 1.

### Stabilization step

According to the invention, a stabilization step may be performed to stabilize the labeled proteins in the sample. With the labeling step of the invention, a Schiff base, or hydrazone, is formed by the reaction of carbonyl groups with the hydrazide (figure 1A). Since said Schiff base is a relatively labile bond reversed by hydrolysis, the invention proposes to convert specifically said Schiff base into an alkylamine bond, for example by using a reducing agent (figure 1B). Once reduced, the bonds are highly stable, increasing the probability of further detection. Preferably, this reduction reaction, or stabilization step, is performed by using a reducing agent such as sodium cyanoborohydride, whose specificity towards the Schiff base may avoid affecting the original aldehyde groups.

Advantageously, according to an embodiment of the invention, at least two samples may be treated according to step (a) before to be separately subjected to differential labeling with at least two fluorescent agents according to step (b), and all the samples are mixed together before step (c).
In another embodiment, at least two samples may be treated according to step (a) before to be separately subjected to differential labeling with at least two fluorescent agents according to step (b), and/or a standard sample is subjected to another fluorescent agent that labels all proteins, and all the samples are mixed together before step (c).

Consequently, different samples, differentially labeled with different spectrally resolvable fluorescent agents, can be compared in a same gel of electrophoresis by using 2D-DIGE technology.

For example, one of the samples is the test sample, another sample is a control sample and optionally a third sample is an internal standard sample.

Said internal standard sample may consist in a pooled-sample mixture, for example prepared by mixing equal protein amounts of both the test and the control samples. The inclusion of a pooled-sample mixture as an internal standard sample further allows for a more complex and statistically powerful experimental design that provides increased statistical confidence using Student's t-test or ANOVA.
Advantageously, both the control sample and the test sample are labeled using two different CyHz. The internal standard sample may then be labeled with a third CyHz, different from the two others.
The CyHz dyes have the same ionic and pH characteristics but absorb and/or fluoresce at different wavelengths, producing different color fluorescence. Gels can be analyzed by scanning at the different wavelength used.

Two examples of experimental designs are provided below for two (Design 1) and three (Design 2) different groups of samples. Of course, more than three groups of different samples can be compared following the same rationale.

### Design 1

Group 1 (Control): samples A1, A2, A3, A4.
Group 2 (Mutant): samples B1, B2, B3, B4.
Internal Standard (IS): Mix of samples A1, A2, A3, A4, B1, B2, B3 and B4.

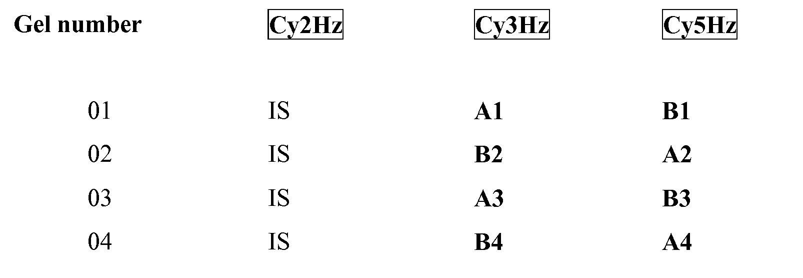

In the first gel, carbonylated proteins of sample A1 are evidenced by a green fluorescent spot, while carbonylated proteins of sample B1 are evidenced by a red fluorescent spot and carbonylated proteins of the internal standard are visualized by a blue fluorescent spot.

In the second gel, carbonylated proteins of sample A2 are evidenced by a red fluorescent spot, while carbonylated proteins of the sample B2 are evidenced by a green fluorescent spot and carbonylated proteins of the internal standard are evidenced by a blue fluorescent spot, etc.

### Design 2

Group 1 (Control): samples A1, A2, A3, A4.
Group 2 (Disease): samples B1, B2, B3, B4.
Group 3 (After therapy): samples C1, C2, C3, C4.
Internal Standard (IS): Mix of samples A1, A2, A3, A4, B1, B2, B3, B4, C1, C2, C3 and C4.

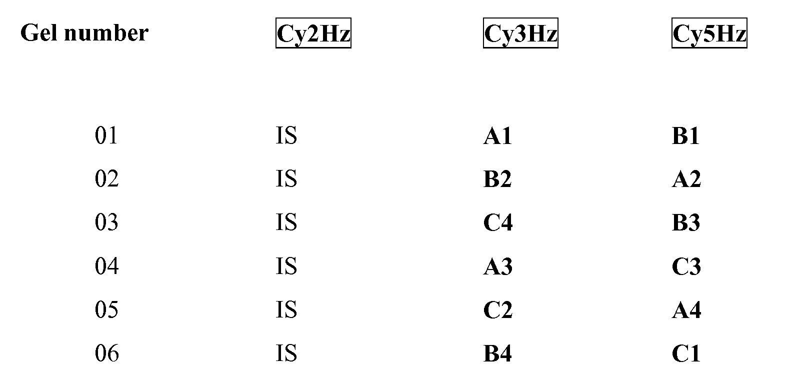

In the first gel, carbonylated proteins of sample A1 are evidenced by green fluorescent spots, while carbonylated proteins of sample B1 are evidenced by red fluorescent spots and carbonylated proteins of the internal standard are visualized by blue fluorescent spots.
In the third gel, carbonylated proteins of sample C4 are evidenced by green fluorescent spots, while carbonylated proteins of sample B3 are evidenced by red fluorescent spots and carbonylated proteins of the internal standard are evidenced by blue fluorescent spots.
In the fourth gel, carbonylated proteins of the sample A3 are evidenced by a green fluorescent spot, while carbonylated proteins of the sample C3 are evidenced by a red fluorescent spot and carbonylated proteins of the internal standard are evidenced by a blue fluorescent spot, etc.

Since the different labeled protein samples are mixed together after labeling and separated by two-dimensional electrophoresis in the same gel (2D-DIGE), the carbonylated proteins present in both samples form coincident spots, evidenced by the merge of the two colors used for labeling the different samples. Carbonylated proteins that are not present in both test and control samples migrate independently and are evidenced by the specific color used for labeling one or the other sample.

Total Protein (carbonylated and non-carbonylated) visualization can be performed, for example, by labeling a separate aliquot of the internal standard, e.g. using a CyDye™ DIGE (NHS Ester) dye, preferably co-resolved with the CyHz labeled samples in the same gel.

The method according to the invention may further comprise a step of removing the denaturing agents and/or fluorescent agent and/or reducing agent that remains free, between step (b) and step (c). This may be achieved by liquid chromatography, preferably by size-exclusion chromatography, but also dialysis could be performed.

### Gel electrophoresis

The labeled proteins are then separated by one or two-dimensional gel electrophoresis, according to their isoelectric point and/or molecular mass.
According to an embodiment of the invention, when different samples are mixed together before performing gel electrophoresis, the labelled proteins are separated by two-dimensional differential in gel electrophoresis (2D-DIGE), according to their isoelectric point and molecular mass. The electrophoresis results in a two-dimensional map of protein spots. Since the protein spots are visualized with fluorescent dyes that bind stoechiometrically to the proteins, a third dimension that corresponds to protein abundance is provided, which facilitates quantitative proteome analysis.

Step (c) is conducted according to standard protocols of one or two-dimensional gel electrophoresis.
If desired, total protein (carbonylated and non-carbonylated) visualization can be performed, by gel post-staining after electrophoresis, e.g. using dyes such as Coomassie Blue or SYPRO™ Ruby stain.

### Image acquisition and data analysis

Step (d) of the method comprises detecting and/or quantifying the labeled carbonyl groups of the proteins resolved by electrophoresis. This may be achieved using standard image acquisition and performing data analysis.
The gel is for example digitized using a multi-wavelength fluorescence scanner like Ettan™ DIGE Imager (GE Healthcare).
Gel analysis as well as statistics and quantification of carbonylated protein spots can be achieved by using commercially available software packages as DeCyder 2-D v7.0 software (GE Healthcare, Inc.).

### Applications

The method of the invention is straightforward. The differences within the samples can be identified in less than 30 min following electrophoresis. Sensitivity and specificity of the method are very good too.
The method is particularly useful for detecting, quantifying, and/or identifying proteins, which carbonylation levels is modified upon ageing or senescence, during a disease, through a mutation, or following a pharmacological treatment or oxidative insult. It can be used also, to screen for potential drugs that are useful for decrease protein carbonylation, and/or to identify proteins affected by carbonylation.

In order to distinguish between changes in protein expression versus changes in the number(s) of carbonylated residues, a traditional 2D-PAGE may be further performed to determine the relative changes at the expression level.

Identification of carbonylation sites may be determined by further mass spectrometry analyses. Indeed, the method of the invention can be coupled with mass fingerprint approaches to identify the differentially carbonylated proteins using mass spectrometry after picking the selected spots from the gels.

The Examples and Figures illustrate the invention without limiting its scope.

### EXAMPLE 1: Comparative study of young and senescent cells

In this example, the endogenous carbonylation of the cellular proteome upon replicative senescence was studied. Protein carbonylation has indeed been documented in senescent fibroblasts.

### PROTOCOL

### a) Preparation of protein extracts (denaturing step):

Human embryonic lung fibroblasts WI-38 (ATCC CCL-75) were replicated until they reached replicative senescence after about 45 cumulative population doublings (cpd). Protein extracts were obtained from four different batches of young (25 cpd) and senescent cells as described below.
Cells were resuspended and homogenized in the corresponding volume of extraction buffer (8M guanidinium hydrochloride, 2M thiourea and 10mM DTT) in order to obtain a protein concentration in the samples (measured by the Bradford method) in the range of 5-10 mg/mL.
After incubation during 10 min on ice, protein extracts were cleaned by centrifugation at 12000xg for 10 min at 4°C.
The pellet was discarded and DNA, lipids, and salts residues were removed from the supernatant by precipitation of proteins with trichloroacetic acid/acetone, using the 2D Clean-Up kit (GE Healthcare) according to manufacturer instructions.
Precipitated proteins were then resuspended in the corresponding volume of extraction buffer in order to obtain a protein concentration in the samples (measured by the Bradford method) in the range of 5-10 mg/mL.
3 to 5 µL of phosphate buffer (1M, pH 7) were added to equilibrate the samples to pH 7.

### b) Labeling and stabilizing step

Protein carbonyls were labeled with the CyDye™ hydrazides (GE Healthcare): Cy3Hz, or Cy5Hz in young and senescent cells.
An internal standard was made by mixing equal amounts of all samples and labeled with HiLyte Fluor™ 488 hydrazide (ANASPEC). 1 mg of each hydrazide derived fluorescent dye was resuspended with 100µl anhydrous dimethyl sulfoxide (DMSO) in order to obtain a 12mM solution and cleaned by centrifugation at 12000xg for 30 seconds.
100 µg of protein samples were incubated with 12 nmol (1 µl) of the corresponding dye (either Cy3Hz or Cy5Hz for samples belonging from young and senescent cells; and HiLyte Fluor™ 488 hydrazide for the internal standard) and 50mM of sodium cyanoborohydride.
Samples were then incubated for 3 hours in the dark at room temperature (about 25°C). Before the isoelectrofocusing, guanidinium hydrochloride and free dyes were removed from the samples using Sephadex G25™ desalting columns previously equilibrated with the isoelectric focusing buffer containing 8M urea, 2M thiourea , 4% (w/v) CHAPS and 10mM dithiothreitol (DTT).

### c) Two-dimensional differential in gel electrophoresis (2D-DIGE)

The three labeled samples (young, senescent and internal standard) in quadruplicates were run on four independent experiments.
The entire assay was performed in the dark.
Pre-cast immobilized pH gradient strips (Immobiline DryStrip 18 cm; pH 3-11 NL) were used for the first dimension separation for a total focusing time of 25 kV-h. First-dimension isoelectric focusing was carried out on a GE Healthcare, Inc. IPGphor 3 IEF system (or any equivalent system).
The strips were then equilibrated with a solution containing 6M urea, 30% (v/v) glycerol, 2% (w/v) sodium dodecyl sulfate (SDS), 50 mM Tris (pH 8.8) supplemented with 10 mM DTT, 4,7% (w/v) iodoacetamide and directly applied to a gradient polyacrylamide gel electrophoresis (8-18% acrylamide-bisacrylamide) for 16h at 60-mA constant current (second dimension).

### d) Image acquisition and data analysis

Gels were scanned using Ettan™ DIGE Imager (GE Healthcare).
The Cy3Hz-labeled gel image was collected at excitation and emission wavelengths of 540/590 nm, the Cy5Hz-labeled gel image at 620/680 nm and the HiLyte Fluor™ 488 hydrazide -labeled gel image at 503/528 nm, respectively.
Gel analysis as well as statistics and quantification of carbonylated protein spots were achieved by using commercially available software packages as DeCyder 2-D v7.0 software (GE Healthcare, Inc.).
The differences within the samples were identified in less than 30 min following electrophoresis.

### RESULTS:

### Step 1: Labeling of carbonylated proteins and resolution of CyHz labeled proteins

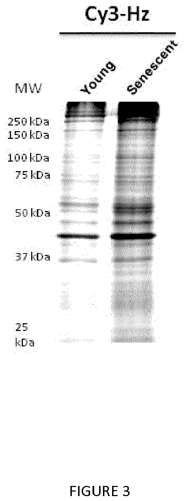

After labeling, samples were resolved by one-dimensional SDS-PAGE to evidence the efficiency of the labeling protocol described above. As depicted in Figure 3, bands corresponding to carbonylated proteins were evidenced with Cy3-Hz, as an example. Therefore, by using the protocol described herein we were able to detect carbonylated proteins.

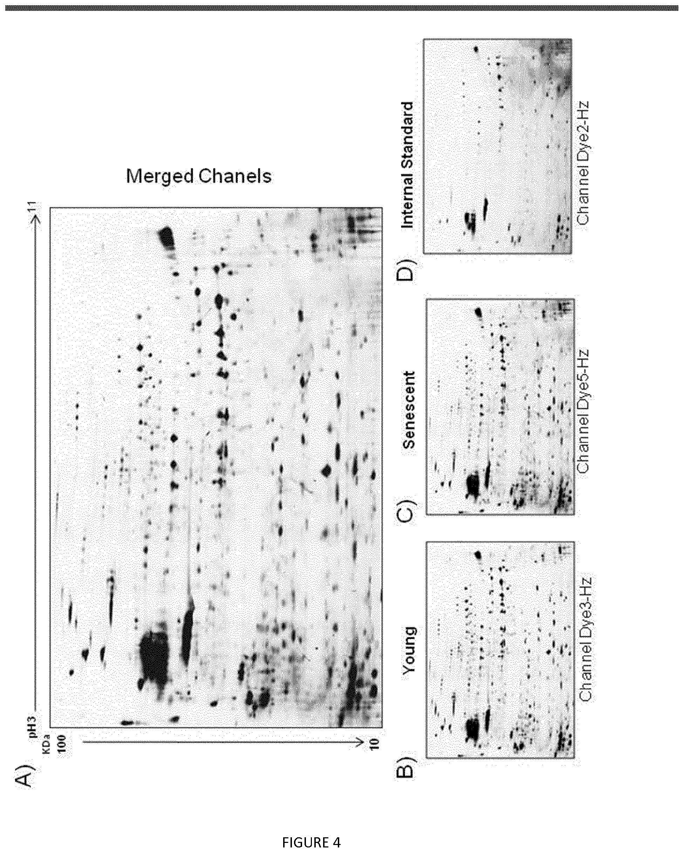

The inventors next performed 2D-DIGE and fluorescent images were obtained. Spots corresponding to carbonylated proteins in all areas of the gels without streaking were evidenced (Figure 4A). The Cy2, Cy3, and Cy5 channels of each gel were individually imaged (Figures 4B-D). Carbonylated proteins in both samples (young and senescent) formed coincident spots, while carbonylated proteins not present in both groups migrated independently. Importantly, a protein that is carbonylated only in young or senescent cells is evidenced by a single color.

### Step 2: Relative quantification of carbonylated protein spots

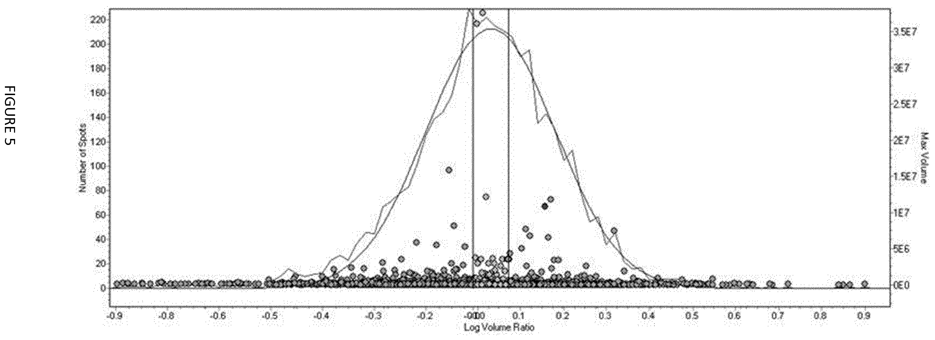

Differential in gel analysis (DIA) as well as biological variation analysis (BVA) were performed to statistically validate the data obtained (Figure 5A). Around 6000 protein spots were evidenced on each gel and around 40% of them were matched in all gels.

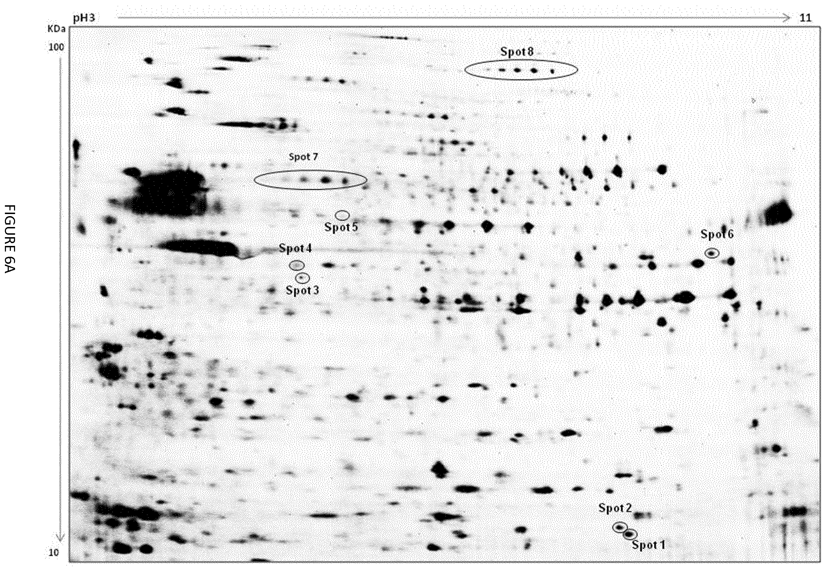

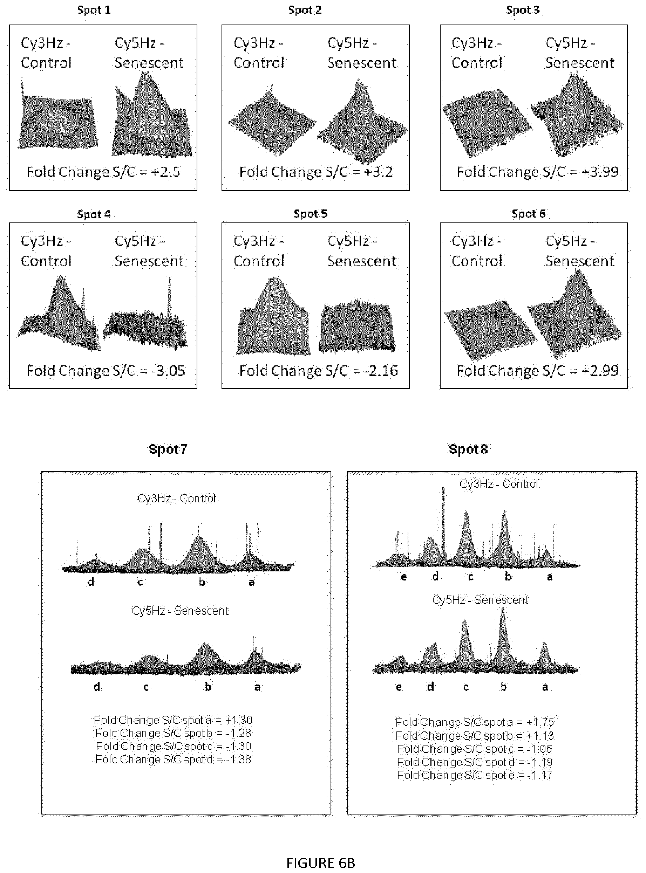

In addition, 3D simulation of the protein spots, allowing an objective view for the comparison of spot intensity between different images was performed using the same analysis software. Some of the spots or train of spots evidenced as differentially carbonylated in senescent and young cells (Figure 6A) were presented in 3D views by its relative amount and distribution (Figure 6B). The comparison of the spot intensities is more objective than with chemioluminiscence in 2D western blot where the brightness/contrast is adjusted manually.

### EXAMPLE 2: Acute Oxidative Stress

In this example, the endogenous carbonylation of the cellular proteome under acute oxidative stress was studied.

### PROTOCOL

Human embryonic lung fibroblasts WI-38 (ATCC CCL-75) were replicated until they reached sub confluence and then treated with 500 µM of hydrogen peroxide for 1 hour in serum free proliferation medium. Protein extracts were obtained; proteins carbonyls were labeled with Cy3Hz and ε-amino group of lysine residues were labeled with Cy5-NHS for total protein visualization (as explained above for example 1). 2D-DIGE was then performed.

### Step 3: Sensitivity and specificity of the method of the invention

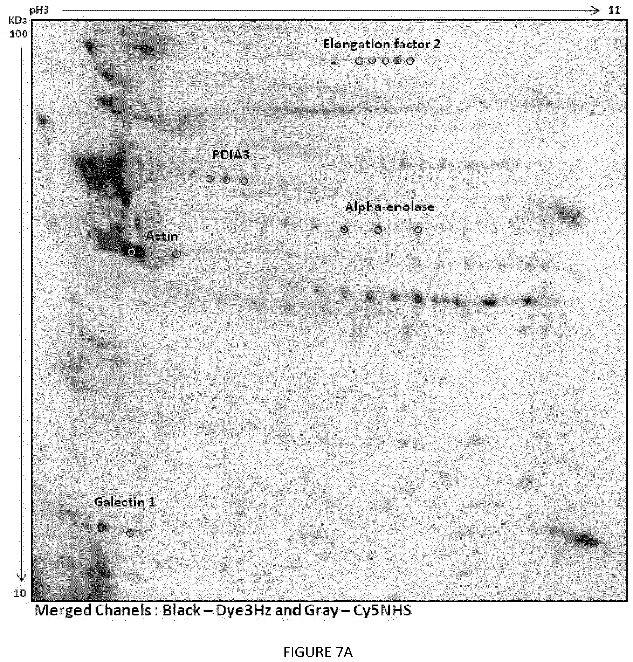

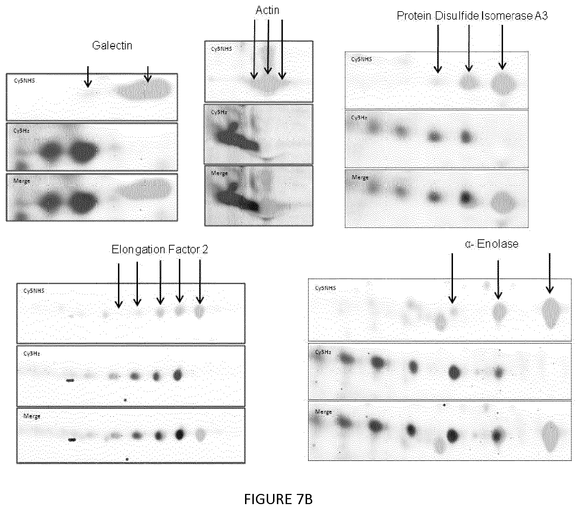

Comparison of the migration pattern of carbonylated proteins labeled with Cy3-Hz or total proteins labeled with Cy5-NHS was performed. Carbonylated proteins (white spots in Figure 7A) were also evidenced in this approach after 2D-DIGE of the invention. Since protein carbonyls introduce negative charge(s) on proteins decreasing their isoelectric point, an alteration of the migration pattern of carbonylated proteins when compared to non-carbonylated proteins after two-dimensional electrophoresis is expected.
As depicted in Figure 7A, the inventors found that a subset of protein spots associated with increased Cy3-Hz fluorescence (and hence, carbonylation) were always shifted to the acidic part of the gel, when compared with the Cy5-NHS labeling (white or grey spots respectively), demonstrating the high sensitivity and specificity reached by using the protocol of the invention.
Also, subsets of train spots were evidenced. These selected spots were excised from gels and further identified by MALDI-TOF/TOF-MS, confirming that the train of spots corresponds to the same protein with increased amounts of modifications (Figure 7B). The proteins identified are known targets of carbonylation.

The 2D-DIGE process of the invention is a selective and sensitive assay to detect endogenous carbonylation of proteins. It provides direct quantification after 2D electrophoresis, which so far has not been possible with any other protein carbonylation assay described to date.
In addition, the method of the invention overcomes inconvenient of the current gel-based methods. In particular, the method of the invention makes it possible to increase accuracy due to: (i) the use of an exogenous synthetic fluorescent tag that reduces false positives; and/or (ii) the possibility to run multiple samples on the same gel, and/or (iii) the use of an internal standard on each gel derived from all proteins which can be run on all gels in a set of experiments. These result in diminishing the effects of gel-to-gel variation.

## Claims

1. A method for detecting and/or quantifying protein carbonyl groups in at least one protein-containing sample comprising the steps of:
(a) subjecting the sample to a protein denaturing agent;
(b) treating the sample with at least a fluorescent agent that is a hydrazide coupled to a fluorescent dye, whereby protein carbonyl groups are labeled;
(c) subjecting the sample to one-dimensional or two-dimensional gel electrophoresis; and
(d) detecting and/or quantifying the labeled carbonyl groups of the proteins resolved by electrophoresis;
wherein the protein denaturing agent is a carbonyl-free denaturing agent selected from guanidine and salt thereof, and is at a concentration between 6M and 8M.

2. The method of claim 1, further comprising an additional step of reducing the Schiff base formed by the hydrazide with the protein carbonyl groups, before step (c).

3. The method of claim 2, wherein the additional step is performed by using a reducing agent, preferably selected among sodium borohydride, and/or sodium cyano borohydride.

4. The method according to any of claims 1 to 3, wherein the fluorescent agent is Cy2Hz, Cy3Hz or Cy5Hz.

5. The method according to any of claims 1 to 4, wherein the sample is not contacted with urea or CHAPS.

6. The method according to any of claims 1 to 5, further comprising, between step (b) and step (c), a step of removing the carbonyl-free denaturing agent, and/or a step of removing fluorescent agent that remains free, and/or a step of removing reducing agent that remains free, said removal(s) being preferably performed by size-exclusion chromatography or dialysis.

7. The method according to any of claims 1 to 6, further comprising a step of removing DNA, lipids and/or salt residues before step (b), preferably by precipitating the proteins.

8. The method according to any of claims 1 to 7, wherein the denaturing agent is guanidine hydrochloride and/or guanidine thiocyanate.

9. The method according to any of claims 1 to 8, wherein the sample in step (a) is contacted with a buffer that comprises thiourea, preferably between 1M to 3M, more preferably 2M.

10. The method according to any of claims 1 to 9, wherein the protein-containing sample is a biological sample.

11. The method according to any of claims 1 to 10, wherein steps (a) and (b) are simultaneous.

12. The method according to any of claims 1 to 11, wherein at least two samples are subjected to a protein denaturing agent, according to step (a), and wherein said at least two samples are separately subjected to differential labeling with at least two fluorescent agents that are hydrazide fluorescent dyes, according to step (b), and/or wherein a standard sample is subjected to another fluorescent agent that labels all proteins, and wherein all the samples are mixed together before step (c).

13. The method according to claim 12, wherein one of the samples is an internal standard sample, which is a pooled-sample mixture of a control and a test samples, labeled with a third fluorescent agent according to step (b).

14. A method for detecting, quantifying, and/or identifying a protein that becomes carbonylated upon ageing or during a disease, through a mutation or after a treatment, in a biological protein-containing test sample, which method comprises the steps of:
(a1) subjecting the test sample to a protein denaturing agent that is carbonyl-free;
(b1) treating the test sample with a fluorescent agent that is a hydrazide coupled with a fluorescent dye whereby protein carbonyl groups are labeled;
separately
(a2) subjecting a reference or control sample to a protein denaturing agent that is carbonyl-free;
(b2) treating the reference or control sample with a different fluorescent agent that produces a different color fluorescence, whereby protein carbonyl groups are labeled;
(c) mixing the two samples and subjecting them to a two-dimensional gel electrophoresis; and
(d) detecting and/or quantifying the differentially labeled carbonyl groups of the proteins resolved by electrophoresis;
(e) optionally identifying the differentially carbonylated proteins, preferably by using mass spectrometer
wherein the protein denaturing agent is a carbonyl-free denaturing agent selected from guanidine and salt thereof, and is at a concentration between 6M and 8M.

15. The method according to claim 14, further comprising an additional step of reducing the Schiff base formed by the hydrazide with the protein carbonyl groups, in at least one sample, before step (c).

## Patentansprüche

1. Ein Verfahren zum Nachweisen und/oder Quantifizieren von Proteincarbonylgruppen in mindestens einer proteinhaltigen Probe, das Verfahren umfasst die Schritte:
(a) Aussetzen der Probe einem Proteindenaturierungsmittel;
(b) Behandeln der Probe mit mindestens einem Fluoreszenzmittel, bei dem es sich um ein Hydrazid handelt, das an einen Fluoreszenzfarbstoff gekoppelt ist, wodurch Proteincarbonylgruppen markiert werden;
(c) Unterziehen der Probe einer eindimensionalen oder zweidimensionalen Gelelektrophorese; und
(d) Nachweisen und/oder Quantifizieren der markierten Carbonylgruppen der Proteine, die durch Elektrophorese aufgetrennt wurden;
wobei das Proteindenaturierungsmittel ein carbonylfreies Denaturierungsmittel ausgewählt aus Guanidin und einem Salz davon ist und in einer Konzentration zwischen 6M und 8M vorliegt.

2. Das Verfahren nach Anspruch 1, ferner umfassend vor Schritt (c) einen zusätzlichen Schritt des Reduzierens der Schiff-Base, die durch das Hydrazid mit den Proteincarbonylgruppen gebildet wird.

3. Das Verfahren nach Anspruch 2, wobei der zusätzliche Schritt unter Verwendung eines Reduktionsmittels durchgeführt wird, vorzugsweise ausgewählt aus Natriumborhydrid und/oder Natriumcyanoborhydrid.

4. Verfahren nach einem der Ansprüche 1 bis 3, wobei das Fluoreszenzmittel Cy2Hz, Cy3Hz oder Cy5Hz ist.

5. Das Verfahren nach einem der Ansprüche 1 bis 4, wobei die Probe nicht mit Harnstoff oder CHAPS in Kontakt gebracht wird.

6. Das Verfahren nach einem der Ansprüche 1 bis 5, ferner umfassend zwischen Schritt (b) und Schritt (c) einen Schritt des Entfernens des carbonylfreien Denaturierungsmittels und/oder einen Schritt des Entfernens des frei bleibenden Fluoreszenzmittels und/oder einen Schritt des Entfernens des frei bleibenden Reduktionsmittels, wobei das Entfernen vorzugsweise durch Größenausschlusschromatographie oder Dialyse durchgeführt wird.

7. Das Verfahren nach einem der Ansprüche 1 bis 6, ferner umfassend vor Schritt (b) einen Schritt des Entfernens von DNA, Lipiden und/oder Salzresten, vorzugsweise durch Ausfällen der Proteine.

8. Das Verfahren nach einem der Ansprüche 1 bis 7, wobei das Denaturierungsmittel Guanidinhydrochlorid und/oder Guanidinthiocyanat ist.

9. Das Verfahren nach einem der Ansprüche 1 bis 8, wobei die Probe in Schritt (a) mit einem Puffer in Kontakt gebracht wird, der Thioharnstoff umfasst, vorzugsweise zwischen 1M bis 3M, noch bevorzugter 2M.

10. Das Verfahren nach einem der Ansprüche 1 bis 9, wobei die proteinhaltige Probe eine biologische Probe ist.

11. Das Verfahren nach einem der Ansprüche 1 bis 10, wobei die Schritte (a) und (b) gleichzeitig stattfinden.

12. Das Verfahren nach einem der Ansprüche 1 bis 11, wobei mindestens zwei Proben einem Proteindenaturierungsmittel gemäß Schritt (a) ausgesetzt werden und wobei die mindestens zwei Proben gemäß Schritt (b) separat einer differentiellen Markierung mit mindestens zwei Fluoreszenzfarbstoffen unterzogen werden, die Hydrazid-Fluoreszenzfarbstoffe sind und/oder wobei eine Standardprobe einem anderen Fluoreszenzmittel ausgesetzt wird, das alle Proteine markiert, und wobei alle Proben vor Schritt (c) zusammengemischt werden.

13. Das Verfahren nach Anspruch 12, wobei eine der Proben eine interne Standardprobe ist, bei der es sich um eine zusammengemischte Probenmischung aus einer Kontroll- und einer Testprobe handelt, die mit einem dritten Fluoreszenzmittel gemäß Schritt (b) markiert ist.

14. Ein Verfahren zum Nachweisen, Quantifizieren und/oder Identifizieren eines Proteins in einer biologischen proteinhaltigen Testprobe, wobei das Protein durch Alterung oder während einer Krankheit, durch eine Mutation oder nach einer Behandlung carbonyliert wird, das Verfahren umfasst die folgenden Schritte:
(a1) Aussetzen der Testprobe einem Proteindenaturierungsmittel, das carbonylfrei ist;
(bl) Behandeln der Testprobe mit einem Fluoreszenzmittel, das ein Hydrazid ist, das mit einem Fluoreszenzfarbstoff gekoppelt ist, wobei die Proteincarbonylgruppen markiert werden;
getrennt
(a2) Aussetzen einer Referenz- oder Kontrollprobe einem Proteindenaturierungsmittel, das carbonylfrei ist;
(b2) Behandeln der Referenz- oder Kontrollprobe mit einem anderen Fluoreszenzmittel, das eine andere Farbfluoreszenz erzeugt, wodurch Proteincarbonylgruppen markiert werden;
(c) Mischen der zwei Proben und Unterwerfen derselben einer zweidimensionalen Gelelektrophorese; und
(d) Nachweisen und/oder Quantifizieren der unterschiedlich markierten Carbonylgruppen der Proteine, die durch Elektrophorese aufgetrennt wurden;
(e) gegebenenfalls Identifizieren der differentiell carbonylierten Proteine, vorzugsweise unter Verwendung eines Massenspektrometers,
wobei das Proteindenaturierungsmittel ein carbonylfreies Denaturierungsmittel ausgewählt aus Guanidin und einem Salz davon ist und in einer Konzentration zwischen 6M und 8M vorliegt.

15. Das Verfahren nach Anspruch 14, ferner umfassend vor Schritt (c) einen zusätzlichen Schritt des Reduzierens der Schiff-Base, die durch das Hydrazid mit den Proteincarbonylgruppen gebildet wird, in mindestens einer Probe.

## Revendications

1. Méthode pour détecter et/ou quantifier les groupes carbonyles de protéines dans au moins un échantillon contenant des protéines comprenant les étapes :
(a) soumettre l'échantillon à un agent dénaturant les protéines ;
(b) traiter l'échantillon avec au moins un agent fluorescent qui est une hydrazide couplée à un colorant fluorescent, les groupes carbonyles des protéines étant ainsi marqués ;
(c) soumettre l'échantillon à une électrophorèse sur gel à une ou deux dimensions ; et
(d) détecter et/ou quantifier les groupes carbonyles marqués des protéines, révélés par l'électrophorèse ;
dans laquelle l'agent dénaturant les protéines est un agent dénaturant sans carbonyle choisi parmi la guanidine et ses sels, et est à une concentration comprise entre 6M et 8M.

2. Méthode selon la revendication 1, comprenant en outre une étape additionnelle de réduction de la base de Schiff formée par l'hydrazide avec les groupes carbonyles des protéines, avant l'étape (c).

3. Méthode selon la revendication 2, dans laquelle 'étape additionnelle est réalisée en utilisant un agent réducteur, préférentiellement sélectionné parmi le borohydrure de sodium et/ou le cyano borohydrure de sodium.

4. Méthode selon l'une quelconque des revendications 1 à 3, dans laquelle l'agent fluorescent est Cy2Hz, Cy3Hz ou Cy5Hz.

5. Méthode selon l'une quelconque des revendications 1 à 4, dans laquelle l'échantillon n'est pas mis en contact d'urée ou de CHAPS.

6. Méthode selon l'une quelconque des revendications 1 à 5, comprenant en outre, entre l'étape b) et l'étape (c), une étape d'élimination de l'agent dénaturant sans carbonyle, et/ou une étape d'élimination de l'agent fluorescent resté libre, et/ou une étape d'élimination de l'agent réducteur resté libre, ladite ou lesdites élimination(s) étant préférentiellement réalisée(s) par chromatographie par exclusion de taille ou dialyse.

7. Méthode selon l'une quelconque des revendications 1 à 6, comprenant en outre une étape d'élimination de résidus d'ADN, lipides et/ou sel, avant l'étape (b), préférentiellement par précipitation de protéines.

8. Méthode selon l'une quelconque des revendications 1 à 7, dans laquelle l'agent dénaturant est le chlorhydrate de guanidine ou le thiocyanate de guanidine.

9. Méthode selon l'une quelconque des revendications 1 à 8, dans laquelle l'échantillon dans l'étape (a) est mis en contact avec un tampon qui comprend de la thiourée, préférentiellement entre 1M et 3M, plus préférentiellement 2M.

10. Méthode selon l'une quelconque des revendications 1 à 9, dans laquelle l'échantillon contenant les protéines est un échantillon biologique.

11. Méthode selon l'une quelconque des revendications 1 à 10, dans laquelle les étapes (a) et (b) sont simultanées.

12. Méthode selon l'une quelconque des revendications 1 à 11, dans laquelle au moins deux échantillons sont soumis à un agent dénaturant les protéines, selon l'étape (a), et dans laquelle au moins deux échantillons sont soumis séparément à un marquage différentiel avec au moins deux agents fluorescents qui sont des colorants hydrazides fluorescents, selon l'étape (b), et/ou dans laquelle un échantillon standard est soumis à un autre agent fluorescent qui marque toutes les protéines, et dans laquelle tous les échantillons sont mélangés ensembles avant l'étape (c).

13. Méthode selon la revendication 12, dans laquelle un des échantillons est un échantillon de standard interne, qui est un mélange d'échantillon groupé d'un échantillon contrôle et d'un échantillon test, marqués avec un troisième agent fluorescent selon l'étape (b).

14. Méthode pour détecter, quantifier et/ou identifier une protéine qui se carbonyles du fait de l'âge ou durant une maladie, par une mutation ou après un traitement, dans un échantillon biologique test contenant des protéines, ladite méthode comprenant les étapes :
(a1) soumettre l'échantillon test à un agent dénaturant les protéines sans carbonyle ;
(b1) traiter l'échantillon test avec un agent fluorescent qui est une hydrazide couplée à un colorant fluorescent, les groupes carbonyles des protéines étant ainsi marqués ;
séparément,
(a2) soumettre un échantillon de référence ou contrôle à un agent dénaturant les protéines sans carbonyle ;
(b2) traiter l'échantillon de référence ou contrôle avec un agent fluorescent différent qui produit une couleur fluorescente différente, les groupes carbonyles des protéines étant ainsi marqués ;
(c) mélanger les deux échantillons et les soumettre à une électrophorèse sur gel à une ou deux dimensions ; et
(d) détecter et/ou quantifier les groupes carbonyles des protéines marqués différemment, révélés par l'électrophorèse ;
(e) optionnellement identifier les protéines carbonylées de manière différente, préférentiellement en utilisant un spectromètre de masse ;
dans laquelle l'agent dénaturant les protéines est un agent dénaturant sans carbonyle choisi parmi la guanidine et ses sels, et est à une concentration comprise entre 6M et 8M.

15. Méthode selon la revendication 14, comprenant en outre une étape additionnelle de réduction de la base de Schiff formée par l'hydrazide avec les groupes carbonyles des protéines, dans au moins un échantillon, avant l'étape (c).
